# EUROPEAN PATENT APPLICATION

(11) **EP 1 227 154 A2**
(43) Date of publication of application: **31.07.2002**
(21) Application number: 02004325.3
(22) Date of filing: 13.12.1993
(51) Int. Cl.: C12N 15/32, C07K 14/325, C12Q 1/68, A01N 63/00

(54) **Bacillus thuringiensis strains active against lepidopteran and coleopteran pests**

(30) Priority: 15.12.1992 US 991073
(62) Divisional of application: 94905931.5
(71) Applicant: Abbott Laboratories, Abbott Park, il. 60064-6050 (US)
(72) Inventor: Liu, Chi-Li, Davis, CA 95616 (US); Adams, Lee Fremont, Davis, CA 95616 (US); Lufburrow, Patricia A., Davis, CA 95616 (US); Thomas, Michael David, Davis, CA 95616 (US)
(74) Representative: Modiano, Guido, Dr.-Ing.

(57) **Abstract**

The invention is related to a biologically pure *Bacillus thuringiensis* (*B*.*t*.)strain active against lepidopteran and coleopteran pests which produces a bipyramidal crystal consisting essentially of a delta-endotoxin having a molecular weight of about 130,000 daltons and a rhomboidal crystal consisting essentially of two delta-endotoxins, each having a molecular weight of about 33,000 daltons, as well as spores, crystals, delta-endotoxins and/or mutants thereof. The invention also relates to insecticidal compositions obstainable therefrom. The invention further relates to methods of using the insecticidal compositions to control insect pest from the order Lepidoptera and/or Coleoptera. The invention also relates to isolated DNA sequences encoding the delta-endotoxins.

SEQUENCE LISTING

(1) GENERAL INFORMATION:

(i) APPLICANT: Liu, Chi-Li
Adams, Lee F.
Lufburrow, Patricia A.
Thomas, Michael D.

(ii) TITLE OF INVENTION: NOVEL BACILLUS THURINGIENSIS STRAINS
ACTIVE AGAINST LEPIDOPTERAN AND COLEOPTERAN PESTS

(iii) NUMBER OF SEQUENCES: 36

(iv) CORRESPONDENCE ADDRESS:
(A) ADDRESSEE: Novo Nordisk of North America, Inc.
(B) STREET: 405 Lexington Avenue
(C) CITY: New York
(D) STATE: New York
(E) COUNTRY: USA
(F) ZIP: 10174-6201

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER:
(B) FILING DATE: 13-DEC-1993
(C) CLASSIFICATION:

(vii) PRIOR APPLICATION DATA:
(A) APPLICATION NUMBER: US 07/991,073
(B) FILING DATE: 15-DEC-1992

(viii) ATTORNEY/AGENT INFORMATION:
(A) NAME: Agris, Cheryl H.
(B) REGISTRATION NUMBER: 34,086
(C) REFERENCE/DOCKET NUMBER: 3778.204-WO

(ix) TELECOMMUNICATION INFORMATION:
(A) TELEPHONE: 212-867-0123
(B) TELEFAX: 212-878-9655

(2) INFORMATION FOR SEQ ID NO:1:

(i) SEQUENCE CHARACTERISTICS:
(A) LENGTH: 26 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:1:

Met Ile Val Asp Leu Tyr Arg Tyr Leu Gly Gly Leu Ala Ala Val Asn
1               5                   10                  15

Ala Val Leu His Phe Tyr Glu Pro Arg Pro
            20                  25

(2) INFORMATION FOR SEQ ID NO:2:

(i) SEQUENCE CHARACTERISTICS:
(A) LENGTH: 10 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:2:

Met Lys His His Lys Asn Phe Asp His Ile
1               5                   10

(2) INFORMATION FOR SEQ ID NO:3:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:3:

CTGCTCCAGC TGCTTGGCTC                                                20

(2) INFORMATION FOR SEQ ID NO:4:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:4:

GAATTATACT TGGTTCAGGC CC                                             22

(2) INFORMATION FOR SEQ ID NO:5:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:5:

GCACACCTTA CATTTTAAAG CA                                             22

(2) INFORMATION FOR SEQ ID NO:6:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:6:

AGATTACAAG CGGATACCAA CATCGCG 27

(2) INFORMATION FOR SEQ ID NO:7:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:7:

TGGCACTTTC AAAATAACCA A 21

(2) INFORMATION FOR SEQ ID NO:8:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:8:

GCATCGGATA GTATTACTCA AATCCC 26

(2) INFORMATION FOR SEQ ID NO:9:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:9:

CGCTCTAACA TAGACCTTAT AA 22

(2) INFORMATION FOR SEQ ID NO:10:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:10:

GACATTTCAT TAGGGCTTAT TAATTT 26

(2) INFORMATION FOR SEQ ID NO:11:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:11:

CAGCGGACGG CCAGACCGCA AG 22

(2) INFORMATION FOR SEQ ID NO:12:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:12:

GTCGGAGTCA ACAACCTTAG GGGC 24

(2) INFORMATION FOR SEQ ID NO:13:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:13:

ATCCGGAAAA GCCGCTATGT C 21

(2) INFORMATION FOR SEQ ID NO:14:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:14:

ATCCGGAAAA GCCGCTATGT C 21

(2) INFORMATION FOR SEQ ID NO:15:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:15:

GGCCAGAAAA TGGAAAAATT TGGG 24

(2) INFORMATION FOR SEQ ID NO:16:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:16:

GTGGGTACAG GAGGTACCAA A 21

(2) INFORMATION FOR SEQ ID NO:17:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:17:

GTGGGTACAG GAGGTACCAA A 21

(2) INFORMATION FOR SEQ ID NO:18:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:18:

CGAAATACTA TGAGTGTAAC TGC 23

(2) INFORMATION FOR SEQ ID NO:19:

(i) SEQUENCE CHARACTERISTICS:
(A) LENGTH: 54 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:19:

YTNGGNGGNY TNGCNGCNGT NAAYGCNGTN YTNCAYTTYT AYGARCCNMG NCCN 54

(2) INFORMATION FOR SEQ ID NO:20:

(i) SEQUENCE CHARACTERISTICS:
(A) LENGTH: 57 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:20:

ATGATGTGAY YTTAYMGTAY YTGGGGYTGC GCGTAAYGCG TYTCAYTTYT AYGARCC 57

(2) INFORMATION FOR SEQ ID NO:21:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:21:

ATGAAACATC ATAAAAATTT TGATCATAT 29

(2) INFORMATION FOR SEQ ID NO:22:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:22:

TTGAATTCAT ATCTACTAAT GAGCAATCGA A 31

(2) INFORMATION FOR SEQ ID NO:23:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:23:

CCACACGCCT AGATTCTCAT GC 22

(2) INFORMATION FOR SEQ ID NO:24:

(i) SEQUENCE CHARACTERISTICS:
(A) LENGTH: 46 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:24:

CGGGATCCAC AGTTACAGTC TGTAGCTCAA TTACCTACTT TTAACG 46

(2) INFORMATION FOR SEQ ID NO:25:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:25:

GGCCAAGGTT GCTGTAATAA TCG 23

(2) INFORMATION FOR SEQ ID NO:26:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:26:

CTCAATATTC TCGAAGCTGG GGCC 24

(2) INFORMATION FOR SEQ ID NO:27:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:27:

GCAGTCTGTA CGGAATTTAT ACA 23

(2) INFORMATION FOR SEQ ID NO:28:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:28:

CGAGGGTTAG CAGATAGCTA TG 22

(2) INFORMATION FOR SEQ ID NO:29:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:29:

AAGATGGGGC GGTCTAACTC C 21

(2) INFORMATION FOR SEQ ID NO:30:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:30:

GACCGTTATC GGGTGAATCT TTAG 24

(2) INFORMATION FOR SEQ ID NO:31:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:31:

TCGGCTGCAC TCTAAATTGT TGAG 24

(2) INFORMATION FOR SEQ ID NO:32:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:32:

TATTGAGTGA ATTATGGGGG AT 22

(2) INFORMATION FOR SEQ ID NO:33:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:33:

ATGTTCTAAA TTCTAACATA TCG 23

(2) INFORMATION FOR SEQ ID NO:34:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:34:

TTATACCTAG ATCCTATTGT TG 22

(2) INFORMATION FOR SEQ ID NO:35:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:35:

TAACATTTCC ACACTTTTCA ATC 23

(2) INFORMATION FOR SEQ ID NO:36:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:36:

AAGGCTAGCG ACTGCTGTC 19

## Description

### 1. FIELD OF THE INVENTION

The invention is related to a novel biologically pure *Bacillus thuringiensis* (*B*.*t*.) strain(s) active against lepidopteran and coleopteran pests which produces a bipyramidal crystal consisting essentially of a delta-endotoxin having a molecular weight of about 130,000 daltons and a rhomboidal crystal consisting essentially of two delta-endotoxins, each having a molecular weight of about 33,000 daltons, as well as spores, crystals, delta-endotoxins and/or mutants thereof. The invention also relates to insecticidal compositions obtainable therefrom. The invention further relates to methods of using the insecticidal compositions to control an insect pest(s) from the order Lepidoptera and/or Coleoptera. The invention also relates to isolated DNA sequences encoding the delta-endotoxins.

### 2. BACKGROUND OF THE INVENTION

Every year, significant portions of the world's commercially important agricultural crops, including foods, textiles, and various domestic plants are lost to pest infestation, resulting in losses in the millions of dollars. Various strategies have been used in attempting to control such pests.

One strategy is the use of broad spectrum pesticides, chemical pesticides with a broad range of activity. However, there are a number of disadvantages to using such chemical pesticides. Specifically, because of their broad spectrum of activity, these pesticides may destroy non-target organisms such as beneficial insects and parasites of destructive pests. Additionally, these chemical pesticides are frequently toxic to animals and humans, and targeted pests frequently develop resistance when repeatedly exposed to such substances.

Another strategy has involved the use of biopesticides, which make use of naturally occurring pathogens to control insect, fungal and weed infestations of crops. Biopesticides are naturally occuring organisms that produce a toxin(s), a substance toxic to the infesting agent which is generally less harmful to non-target organisms and the environment as a whole than chemical pesticides.

The most widely used biopesticide is *Bacillus thuringiensis* (*B*.*t*.). *B*.*t*. is a widely distributed, rod shaped, aerobic and spore forming microorganism. During its sporulation cycle, *B*.*t*. produces a protein(s) known as a delta-endotoxin(s), that forms crystalline inclusion bodies within the cell. The delta-endotoxins have molecular weights ranging from 27-140 kD and kill insect larvae upon ingestion.

Delta-endotoxins have been produced by recombinant DNA methods (see, for example, Tailor et al., 1992, Molecular Microbiology 6:1211-1217; toxin is active against lepidopteran and coleopteran pests); Payne et al., U.S. Patent No. 5,045,469; toxin is active against lepidopteran pests). The delta-endotoxins produced by recombinant DNA methods may or may not be in crystal form.

A number of *B.t*. strains have been isolated that have been found to be active against insect pests of the order Lepidoptera. *B.t.* subsp. *kurstaki* HD-1 produces bipyramidal and cuboidal crystal proteins in each cell during sporulation (Lüthy et al., in Microbial and Viral Pesticides, ed. E. Kurstak, Marcel Dekker, New York, 1982, pp. 35-74); the bipyramidal crystal was found to be encoded by three *cryIA* genes (Aronson et al., 1986, Microbiol. Rev. 50:1-50). *B*.*t*. subsp. *kurstaki* HD-73 contains the CryIA(c) protein (Adang et al., 1985, Gene 36:289-300). *B*.*t*. *subsp*. *dendrolimus* HD-7 and HD-37 contain a CryIA and a CryII protein; *B.t.* subsp. *sotto* contains an alkaline soluble protein that differs from the holotype CryIA(a) protein by 24 amino acids; *B.t*. subsp. *subtoxicus* HD-10 contains CryIA and CryIB proteins; *B.t*. subsp. *tolworthi* HD-121 contains CryIA and CryII proteins; and *B.t*. subsp. *aizawai* HD-68 contains CryIA proteins (Höfte and Whiteley, 1989, Microbiol. Reviews 53:242-255). Payne, U.S. Patent No. 4,990,332, issued February 5, 1993, discloses an isolate of *B.t*. PS85AI and a mutant of the isolate, PS85AI which both have activity against *Plutella xylostella,* a lepidopteran pest and produce alkaline soluble proteins having a molecular weight of 130,000 and 60,000 daltons. Payne, U.S. Patent No. 5,045,469, issued September 3, 1991 discloses a *B.t*. isolate designated PS81F which also produces alkaline soluble proteins having a molecular weight of 130,000 and 60,000 daltons and has activity against *Spodoptera exigua and T.* ni; the toxin gene from PS81F appears to have little homology to the toxin gene from *B.t*. subsp. *kurstaki* HD-1. Payne, U.S. Patent No. 5,206,166, filed June 25, 1992, issued April 27, 1993, discloses *B.t.* isolates PS81A2 and PS81RR1 which produce 133,601 and 133,367 dalton alkaline-soluble proteins; both have activity against *Trichoplusia ni, Spodoptera exigua* and *Plutella xylostella* and are different from *B.t.* subsp. *kurstaki* HD-1 and other *B.t*. isolates. Bernier et al., U.S. Patent No. 5,061,489 and WO 90/03434 discloses strain A20 producing a delta-endotoxin encoded by at least three genes: 6.6-, 5.3-, and 4.5-type genes (*cryIA(a), cryIA(b), and CryIA(c)).* Chestukhina et al., 1988, FEBS Lett. 232:249-51, discloses that *B*.*t*. subsp. *galleriae* produces two delta-endotoxins, both of which are active against lepidopteran pests.

Other strains, e.g. *Bacillus thuringiensis* subsp. *tenebrionis* (Krieg et al., 1988, U.S. Patent No. 4,766,203), have been found to be specific for Coleoptera. The isolation of another coleopteran toxic *Bacillus thuringiensis* strain was reported in 1986 (Hernnstadt et al. Bio/Technology vol. 4, 305-308, 1986, US patent 4,764,372, 1988). This strain, designated *"Bacillus thuringiensis* subsp. *san diego*", M-7, has been deposited at the Northern Regional Research Laboratory, USA under accession number NRRL B-15939. However, the assignee of the '372 patent, Mycogen, Corp. has publicly acknowledged that *Bacillus thuringiensis* subsp. *san diego* is *Bacillus thuringiensis* subsp. *tenebrionis.*

Other isolated strains have been found to be active against two orders of pests. Padua, 1990, Microbiol. Lett. 66:257-262, discloses the isolation of two mutants containing two delta-endotoxins, a 144 kD protein having activity against a lepidopteran pest and a 66 kD protein having activity against mosquitoes. Bradfish et al., U.S. Patent No. 5,208,017, discloses *B.t.* isolates PS86A1 and PS86Q3 which respectively, produce alkaline soluble proteins having a molecular weight of 58,000 and 45,000 daltons and 155,000, 135,000, 98,000, 62,000, and 58,000 daltons, respectively and which have activity against lepidopteran and coleopteran pests. PCT Application No. WO 90/13651 and Tailor et al., 1992, Molecular Microbiology 6:1211-1217, discloses a *B.t.* strain which is toxic against Lepidoptera and Coleoptera and which produces a toxin having a molecular weight of 81 kd.

It is advantageous to isolate new strains of *Bacillus thuringiensis* to produce new toxins so that there exists a wider spectrum of biopesticides for any given insect pest.

### 3. SUMMARY OF THE INVENTION

The invention is related to a novel biologically pure *Bacillus thuringiensis* strain(s) or a spore(s), crystal(s) or mutant(s) thereof which strain or mutant in contrast to *B.t*. strains disclosed in the prior art, has activity against an insect pest of the order Lepidoptera and an insect pest of the order Coleoptera, produces a delta-endotoxin having a molecular weight of about 130,000 daltons (hereinafter referred to as the "130,000 dalton delta-endotoxin") and two delta-endotoxins both having molecular weights of about 33,000 daltons (hereinafter referred to as the "33,000 dalton delta-endotoxins"). One of the 33,000 dalton delta-endotoxins has an N-terminal amino acid sequence of MIVDLYRYLGGLAAVNAVLHFYEPRP (SEQ ID NO:1). The other 33,000 dalton delta-endotoxin has an N-terminal amino acid sequence of

MKHHKNFDHI (SEQ ID NO:2).

The invention also relates to each of the delta-endotoxins as well as a nucleic acid fragment containing a nucleic acid sequence encoding each of the delta-endotoxins or a portion of the delta-endotoxin having insecticidal activity against a lepidopteran and/or coleopteran pest. The 130,000 and 33,000 dalton delta-endotoxins singly have activity against lepidopteran pests and together have insecticidal activity against lepidopteran and coleopteran pests. The delta-endotoxins may optionally be in crystalline form; the 130,000 dalton delta-endotoxin is bipyramidal and the 33,000 dalton delta-endotoxins are rhomboidal.

In a specific embodiment of the invention, the *thuringiensis* strain of the present invention is EMCC0075 and EMCC0076 having the identifying characteristics of NRRL B-21019 and NRRL B-21020 respectively.

The novel *Bacillus thuringiensis* strains, spores, mutants or crystals and/or delta-endotoxins may within the scope of this invention each be formulated into insecticidal compositions. In one embodiment, the strain, spores, mutants, crystals, and/or delta-endotoxins may be combined with an insecticidal carrier. Insecticidal compositions comprising the strains or mutants of the invention and/or spores, and/or crystals thereof may be used to control insect pests of the order Lepidoptera and and/or insect pests of the order Coleoptera in a method comprising exposing the pest to an insect-controlling effective amount of such an insecticidal composition.

### 4. BRIEF DESCRIPTION OF THE FIGURES

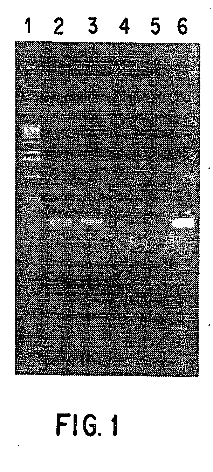

Figure 1 shows the results of PCR analysis of *Bacillus thuringiensis* strains for *cryI* genes by agarose gel electrophoresis. Lane 1 shows molecular weight markers (1 kb ladder, BRL-GIBCO). Lanes 2 and 3 show analysis of strains EMCC0075 and EMCC0076 with *cryID* oligonucleotide primers described in Figure 1. Lanes 4-6 show the analysis of *Bacillus thuringiensis* subsp. *tenebrionis*, an unknown *Bacillus thuringiensis* strain, and *Bacillus thuringiensis* subsp. *aizawai* with *cryID* oligonucleotide primers. *Bacillus thuringiensis* subsp. *tenebrionis* contains only the *cryIIIA* gene; the unknown *Bacillus thuringiensis* strain does not contain the *cryJD* gene; and *Bacillus thuringiensis* subsp. *aizawai* contains several *cryI* genes including *cryID*.

### 5. DETAILED DESCRIPTION OF THE INVENTION

### 5.1. OBTAINING DELTA-ENDOTOXINS

The spores and crystals of the present invention are obtainable from the strains of the present invention. The strains of the present invention may be cultured using media and fermentation techniques known in the art (see, for example, Rogoff et al., 1969, J. Invertebrate Path. 14:122-129; Dulmage et al., 1971, J. Invertebrate Path. 18:353-358; Dulmage et al., in Microbial Control of Pests and Plant Diseases, H.D. Burges, ed., Academic Press, N.Y., 1980). Upon completion of the fermentation cycle, the crystals and spores can be harvested by separating B.t. spores and crystals from the fermentation broth by means well known in the art, e.g. centrifugation. The spores and crystals are contained in the pellet.

As noted in Section 2, *supra*, crystals consist essentially of a delta-endotoxin(s). The strains of the present invention produce two types of crystals. One is a bipyramidal crystal consisting essentially of the 130,000 dalton delta-endotoxin. The other is a rhomboidal crystal consisting essentially of the two 33,000 dalton delta-endotoxins.

Purification of the crystals or delta-endotoxins can be carried out by various procedures known in the art, including but not limited to density gradient centrifugation, chromatography (e.g. ion exchange, affinity, hydrophobic and size exclusion), electrophoretic procedures, differential solubility, or any other standard technique for the purification of proteins.

The delta-endotoxins may also be obtained from a recombinant DNA expression system. Specifically, DNA encoding each toxin is cloned into a suitable DNA expression vector.

Identification of the specific DNA fragment encoding the delta-endotoxin may be accomplished in a number of ways, including, but not limited to, electrophoretic separation of the fragments (Southern, 1975, J. Mol. Biol. 98:503)in agarose, transfer of the separated DNA fragments to nitrocellulose, nylon, or other suitable support medium, and probing of the transferred fragments with a degenerate oligonucleotide probe(s) based on the amino acid sequence of the protein as determined by sequential Edman degradation. Alternatively, one may probe with a labeled gene fragment corresponding to the open reading frame of a protein with suspected high homology to the protein of interest. High homology to the gene of interest may be determined by alignment of a family of related proteins and identification of highly conserved regions in the encoding DNA segments (see, for example, Gribskov, K., and J. Devereux, eds., in Sequence Analysis Primer, Stockton Press, N.Y., 1991). An elegant and reliable method is to determine the amino acid sequences of at least two peptide fragments, generated by enzymatic or chemical means from the protein of interest, design degenerate oligonucleotides that will recognize the DNA encoding those regions, and then applying polymerase chain reaction (PCR) techniques to amplify perfect or near-perfect copies of the intervening region of DNA. This PCR-generated segment of DNA can then be labeled and used as a highly specific probe for cloning the delta-endotoxin-encoding gene.

Once identified, the DNA fragment harboring the gene encoding the delta-endotoxin or a portion thereof may be cloned by ligation of a size-selected library of fragments expected to harbor the gene of interest into a suitable vector, including, but is not limited to, pBR322, pUC118, pACYC194, and pBCSK plasmids and their variants for transformation into *Escherichia coli;* or pUB110, pBD64, pBC16, pHP13, pE194, pC194, and their variants, for transformation into *Bacillus* spp. Bacteriophage vectors, such as lambda and its derivatives, may also be used for cloning of the gene(s) into *E*. *coli*.

Production of the delta-endotoxin or a portion thereof at commercially useful levels can be achieved by subcloning the encoding gene into plasmid vectors that permit stable expression and maintenance in a suitable host. Frequently, acceptable expression can be achieved using the native regulatory elements present on the DNA fragment encoding the delta-endotoxin. However, one might wish to add or alter transcriptional regulatory signals (promoters, initiation start sites, operators, activator regions, terminators) and translational regulatory signals (ribosomal binding sites, initiation codons) for enhanced or more regulated expression of the delta-endotoxin gene within the chosen host cell.

In addition to plasmids, delta-endotoxin genes and the appropriate regulatory elements may be introduced into one of the native plasmids of *Bacillus thuringiensis* and/or other chosen host, or into the chromosomal DNA, via "gene conversion" (e.g., Iglesias and Trautner, 1983, Mol Gen. Genet. 189:73-76; Duncan et al., 1978, Proc. Natl. Acad. Sci. U.S.A. 75:3664-3665) or homologous recombination (e.g., Ferrari et al., 1983, J. Bacteriol. 154:1513-1515) at sites of shared DNA homology between the vector and the host strain. An efficient "two-plasmid" system may be used for introduction of genes into Bacilli via homologous recombination (see, for example, PCT Patent WO91/09129). Transposons may also be used to introduce *cry* genes into the selected host strain. For example, in the Bacilli, transposons such as Tn917 and its derivatives may be used (Youngman et al., 1989, *In* Regulation of Prokaryotic Development, I. Smith, R. Slepecky, and P. Setlow, eds. American Society for Microbiology, Washington, D.C.).

Transfer of cloned delta-endotoxin genes into *Bacillus thuringiensis*, as well as into other organisms, may be achieved by a variety of techniques, including, but not limited to, protoplasting of cells (Chang and Cohen, 1979, Mol. Gen. Genet. 168: 111-115; Crawford et al., 1987, J. Bacteriol. 169: 5423-5428); electroporation (e.g., Schurter et al., 1989, Mol. Gen. Genet. 218: 177-181 and Macaluso et al., 1991, J. Bacteriol. 173: 1353-1356); particle bombardment (e.g., Shark et al., 1991, Appl. Environ. Microbiol. 57:480-485); silicon carbide fiber-mediated transformation of cells (Kaeppler et al., 1992, Theor. Appl. Genet. 84:560-566); conjugation (Gonzalez et al., 1982, Proc. Natl. Acad. Sci. U.S.A. 79:6951-6955); or transduction by bacteriophage (e.g., Lecadet et al., 1992, Appl. Environ. Microbiol. 58: 840-849). Transformed colonies may be detected by their ability to produce crystal delta-endotoxin, to bind antibody directed against that specific delta-endotoxin, or to kill susceptible pests, e.g., arthropods or nematodes, in bioassay.

Criteria for selection of a particular host for production include, but are not limited to, ease of introducing the gene into the host, availability of expression systems, and stable maintenance and expression of the gene encoding the delta-endotoxin. The host may be a microorganism, such as *Bacillus thuringiensis* itself, or an inhabitant of the phytosphere, e.g., the phylloplane (the surface of plants), and/or the rhizosphere (the soil surrounding plant roots), and/or aquatic environments, and should be capable of competing in the particular environment (crop and other insect habitats) with the wild-type microorganisms. Examples of such microorganisms include but are not limited to bacteria, e.g. genera *Bacillus, Pseudomonas, Erwinia, Serratia, Klebsiella, Xanthomonas, Streptomyces, Rhizobium, Rhodopseudomonas, Methylophilius, Agrobacterium, Acetobacter, Lactobacillus, Arthrobacter, Azotobacter, Leuconostoc, Alcaligenes*, and *Clostridium*; algae, e.g. families *Cyanophyceae, Prochlorophyceae, Rhodophyceae, Dinophyceae, Chrysophyceae, Prymnesiophyceae, Xanthophyceae, Raphidophyceae, Bacillariophyceae, Eustigmatophyceae, Cryptophyceae, Euglenophyceae, Prasinophyceae,* and *Chlorophyceae;* and fungi, particularly yeast, e.g. genera *Saccharomyces, Cryptococcus, Kluyveromyces, Sporobolomyces, Rhodotorula,* and *Aureobasidium.*

The gene(s) encoding the delta-endotoxin(s) of the present invention or a portion thereof can also be inserted into an appropriate cloning vector for subsequent introduction into the genomes of suitable plants that are known to be infested with insects susceptible to the delta-endotoxin(s), or into specific baculoviruses which can in turn be directly used as insecticides.

### 5.2. MUTANTS

The invention is also directed to a mutant *B*.*t*. strain which produces a larger amount of and/or larger crystals than the parental strain. A "parental strain" as defined herein is the original *Bacillus thuringiensis* strain before mutagenesis.

To obtain such mutants, the parental strain may, for example, be treated with a mutagen by chemical means such as N-methyl-N'-nitro-N-nitrosoguanidine or ethyl methanesulfonate, gamma-irradiation, X-ray or UV-irradiation. Specifically, in one method of mutating *Bacillus thuringiensis* strains and selecting such mutants the following procedure is used:
i) the parental strain is treated with a mutagen;
ii) the thus presumptive mutants are grown in a medium suitable for the selection of a mutant strain; and
iii) the mutant strain is selected.

According to a preferred embodiment of this method, the selected colonies are grown in a normal production medium, and a final selection for strains capable of increased delta-endotoxin production is performed.

Alternatively, the mutant(s) may be obtained using recombinant DNA methods known in the art. For example, a DNA sequence containing a gene coding for a delta-endotoxin may be inserted into an appropriate expression vector and subsequently introduced into the parental strain using procedures known in the art. Alternatively, a DNA sequence containing a gene coding for a delta-endotoxin may be inserted into an appropriate vector for recombination into the genome and subsequent amplification.

### 5.3. BIOASSAY

The activity of the *B*.*t*. strains of the present invention or spores, mutants, crystals, or delta-endotoxins thereof against various insect pests may be assayed using procedures known in the art, such as an artificial insect diet incorporation assay, artificial diet overlay, leaf painting, leaf dip, and foliar spray. Specific examples of such assays are given in Section 6, *infra.*

### 5.4. COMPOSITIONS

The strains, spores, crystals, delta-endotoxins, or mutants of the present invention described *supra* can be formulated with an acceptable carrier into an insecticidal composition(s) that is, for example, a suspension, a solution, an emulsion, a dusting powder, a dispersible granule, a wettable powder, an emulsifiable concentrate, an aerosol or impregnated granule.

Such compositions disclosed above may be obtained by the addition of a surface active agent, an inert carrier, a preservative, a humectant, a feeding stimulant, an attractant, an encapsulating agent, a binder, an emulsifier, a dye, a U.V. protectant, a buffer, a flow agent, or other component to facilitate product handling and application for particular target pests.

Suitable surface-active agents include but are not limited to anionic compounds such as a carboxylate, for example, a metal carboxylate of a long chain fatty acid; an N-acylsarcosinate; mono or di-esters of phosphoric acid with fatty alcohol ethoxylates or salts of such esters; fatty alcohol sulphates such as sodium dodecyl sulphate, sodium octadecyl sulphate or sodium cetyl sulphate; ethoxylated fatty alcohol sulphates; ethoxylated alkylphenol sulphates; lignin sulphonates; petroleum sulphonates; alkyl aryl sulphonates such as alkyl-benzene sulphonates or lower alkylnaphthalene sulphonates, e.g. butyl-naphthalene sulphonate; salts of sulphonated naphthalene-formaldehyde condensates; salts of sulphonated phenol-formaldehyde condensates; or more complex sulphonates such as the amide sulphonates, e.g. the sulphonated condensation product of oleic acid and N-methyl taurine or the dialkyl sulphosuccinates, e.g. the sodium sulphonate or dioctyl succinate. Non-ionic agents include condensation products of fatty acid esters, fatty alcohols, fatty acid amides or fatty-alkyl- or alkenyl-substituted phenols with ethylene oxide, fatty esters of polyhydric alcohol ethers, e.g. sorbitan fatty acid esters, condensation products of such esters with ethylene oxide, e.g. polyoxyethylene sorbitar fatty acid esters, block copolymers of ethylene oxide and propylene oxide, acetylenic glycols such as 2,4,7,9-tetraethyl-5-decyn-4,7-diol, or ethoxylated acetylenic glycols. Examples of a cationic surface-active agent include, for instance, an aliphatic mono-, di-, or polyamine as an acetate, naphthenate or oleate; an oxygen-containing amine such as an amine oxide of polyoxyethylene alkylamine; an amide-linked amine prepared by the condensation of a carboxylic acid with a di- or polyamine; or a quaternary ammonium salt.

Examples of inert materials include but are not limited to inorganic minerals such as kaolin, phyllosilicates, carbonates, sulfates, phosphates or botanical materials such as cork, powdered corncobs, peanut hulls, rice hulls, and walnut shells.

The compositions of the present invention can be in a suitable form for direct application or as a concentrate or primary powder which requires dilution with a suitable quantity of water or other diluent before application. The insecticidal concentration will vary depending upon the nature of the particular formulation, specifically, whether it is a concentrate or to be used directly. The compositipn contains 1 to 98% of a solid or liquid inert carrier, and 0 to 50%, preferably 0.1 to 50% of a surfactant. These compositions will be administered at the labeled rate for the commercial product, preferably about 0.01 lb-5.0 lb per acre when in dry form and at about 0.01 pts-10 pts per acre when in liquid form.

In a further embodiment, the strains, spores, crystals, delta-endotoxins or mutants of the present invention can be treated prior to formulation to prolong the pesticidal activity when applied to the environment of a target pest as long as the pretreatment is not deleterious to the crystal delta-endotoxin. Such treatment can be by chemical and/or physical means as long as the treatment does not deleteriously affect the properties of the composition(s). Examples of chemical reagents include but are not limited to halogenating agents; aldehydes such as formaldehyde and glutaraldehyde; anti-infectives, such as zephiran chloride; alcohols, such as isopropranol and ethanol; and histological fixatives, such as Bouin's fixative and Helly's fixative (see, for example, Humason, Animal Tissue Techniques, W.H. Freeman and Co., 1967).

The compositions of the invention can be applied directly to the plant by, for example, spraying or dusting at the time when the pest has begun to appear on the plant or before the appearance of pests as a protective measure. Plants to be protected within the scope of the present invention include but are not limited to cereals (wheat, barley, rye, oats, rice, sorghum and related crops), beets (sugar beet and fodder beet), drupes, pomes and soft fruit (apples, pears, plums, peaches, almonds, cherries, strawberries, raspberries, and blackberries), leguminous plants (alfalfa, beans, lentils, peas, soybeans), oil plants (rape, mustard, poppy, olives, sunflowers, coconuts, castor oil plants, cocoa beans, groundnuts), cucumber plants (cucumber, marrows, melons), fibre plants (cotton, flax, hemp, jute), citrus fruit (oranges, lemons, grapefruit, mandarins), vegetables (spinach, lettuce, asparagus, cabbages and other brassicae, carrots, onions, tomatoes, potatoes, paprika), lauraceae (avocados, cinnamon, camphor), deciduous trees and conifers (e.g. linden-trees, yew-trees, oak-trees, alders, poplars, birch-trees, firs, larches, pines), or plants such as maize, turf plants, tobacco, nuts, coffee, sugar cane, tea, vines hops, bananas and natural rubber plants, as well as ornamentals. In most cases, the preferred mode of application is by foliar spraying. The preferred mode of application for soil pests is by furrow application or by "lay-by" application.It is generally important to obtain good control of pests in the early stages of plant growth as this is the time when the plant can be most severely damaged. The spray or dust can conveniently contain another pesticide if this is thought necessary. In a preferred embodiment, the composition of the invention is applied directly to the plant.

The compositions of the present invention may be effective against pests including but not limited to pests of the order Lepidoptera, e.g. *Achroia grisella, Acleris gloverana, Acleris variana, Adoxophyes orana, Agrotis ipsilon, Alabama argillacea, Alsophila pometaria, Amyelois transitella, Anagasta kuehniella, Anarsia lineatella, Anisota senatoria, Antheraea pernyi, Anticarsia gemmatalis, Archips sp., Argyrotaenia sp., Athetis mindara, Bombyx mori, Bucculatrix thurberiella, Cadra cautella, Choristoneura sp., Cochylls hospes, Colias eurytheme, Corcyra cephalonica, Cydia latiferreanus, Cydia pomonella, Datana integerrima, Dendrolimus sibericus, Desmia funeralis, Diaphania hyalinata, Diaphania nitidalis, Diatraea grandiosella, Diatraea saccharalis, Ennomos subsignaria, Eoreuma loftini, Ephestia elutella, Erannis tilaria, Estigmene acrea, Eulia salubricola, Eupocoellia ambiguella, Eupoecilia ambiguella, Euproctis chrysorrhoea, Euxoa messoria, Galleria mellonella, Grapholita molesta, Harrisina americana, Hellcoverpa subflexa, Helicoverpa zea, Heliothis virescens, Hemileuca oliviae, Homoeosoma electellum, Hyphantria cunea, Keiferia lycopersicella, Lambdina fiscellaria fiscellaria, Lambdina fiscellaria lugubrosa, Leucoma salicis, Lobesia botrana, Loxostege sticticalis, Lymantria dispar, Macalla thyrsisalis, Malacosoma sp., Mamestra brassicae, Mamestra configurata, Manduca quinquemaculata, Manduca sexta, Maruca testulalis, Melanchra picta, Operophtera brumata, Orgyia sp., Ostrinia nubilalis, Paleacrita vernata, Papilio cresphontes, Pectinophora gossypiella, Phryganidia californica, Phyllonorycter blancardella, Pieris napi, Pieris rapae, Plathypena scabra, Platynota flouendana, Platynota stultana, Platyptilia carduidactyla, Plodia interpunctella, Plutella xylostella, Pontia protodice, Pseudaletia unipuncta, Pseudoplasia includens, Sabulodes aegrotata, Schizura* *concinna, Sitotroga cerealella, Spilonota ocellana, Spodoptera sp., Thaurnstopoea pityocampa, Tineola bisselliella, Trichoplusia ni, Udea rubigalis, Xylomyges curialis, Yponomeuta padella* and/or *pests of the order* Coleoptera, e.g. *Leptinotarsa sp., Acanthoscelides obtectus, Callosobruchus chinensis, Epilachna varivestis, Pyrrhalta luteola, Cylas formicarius elegantulus, Listronotus oregonensis, Sitophilus sp., Cyclocephala borealis, Cyclocephala immaculata, Macrodactylus subspinosus, Popillia japonica, Rhizotrogus majalis, Alphitoblus diaperinus, Palorus ratzeburgi, Tenebrio molitor, Tenebrio obscurus, Tribolium castaneum, Tribolium confusum, Tribolius destructor.*

In specific embodiments, a composition comprising the 130,000 dalton delta-endotoxin and/or the two 33,000 dalton delta-endotoxins is effective against lepidopteran pests. Compositions comprising the strains and/or spores of the present invention, or the 130,000 dalton delta endotoxin and the two 33,000 dalton delta-endotoxins are active against lepidopteran and coleopteran pests.

The following examples are presented by way of illustration, not by way of limitation.

### 6. EXAMPLES

### 6.1. EXAMPLE 1: CULTIVATING B.t. STRAINS EMCC0075 AND EMCC0076

Subcultures of EMCC0075 and EMCC0076, maintained on Nutrient Broth Agar slants, are used to inoculate 250 ml baffled shake flasks containing 50 ml of medium with the following composition:

| | |
|---|---|
| Corn Steep liquor | 15 g/l |
| Maltrin-100 | 40 g/l |
| Potato Starch | 30 g/l |
| KH₂PO₄ | 1.77 g/l |
| K₂HPO₄ | 4.53 g/l |

The pH of the medium is adjusted to 7.0 using 10 N NaOH.

After inoculation, shake flasks are incubated at 30°C on a rotary shaker with 250 rpm shaking for 72 hours. The *B.t.* crystals, obtained in the above fermentation, are recovered by centrifugation at 15,000 rpm for 15 minutes using a Sorvall RC-5B centrifuge.

### 6.2. EXAMPLE 2: TESTING OF B.t. STRAINS EMCC0075 AND EMCC0076 SPORES AND CRYSTALS

EMCC0075 and EMCC0076 are cultivated in shake flasks as described in Example 1, *supra.* To determine if EMCC0075 and EMCC0076 are active against lepidopteran pests, a 1:50 dilution of culture broth is made. 5 ml of such diluted culture broth is transferred into a 50 ml polypropylene centrifuge tube. 20 ml of artificial insect diet containing antibiotics is added into the centrifuge tube. The mixture is subsequently dispensed into bioassay trays. Three to six eggs either of beet armyworm (*Spodoptera exigua*) or tobacco budworm (*Heliothis virescens)* are applied on the surface of the "diet". Mylar is ironed onto the bioassay trays and the trays are incubated at 28°C. Scoring is carried out at 7 and 11 days.

To determine if EMCC0075 and EMCC0076 are active against insect pests of the order Coleoptera, 5 ml of the culture broths are removed from the shake flasks and transferred directly into the 50 ml polypropylene centrifuge tubes. 20 ml of artificial insect diet (containing known antibiotics) are then added into the tubes (final testing concentration=20 % w/w) and mixed vigorously. The mixtures are then dispensed into bioassay trays. Three to six eggs of corn rootworm (*Diabrotica undecimpunctata*) are applied to the surface of the "diet". Mylar is ironed onto the bioassay trays and the trays are incubated at 28°C. Scoring is carried out at 7 and 11 days.

The bioactivity of EMCC0075 and EMCC0076 towards *Spodoptera exigua* and *Diabrotica undecimpunctata* is expressed in terms of stunt score (SS). The stunt score is determined after incubating the trays for 7 days. In this system, 4=full size larvae (control larvae); 3=3/4 size of control larvae; 2=1/2 size'of control larvae; 1= 1/4 size of control larvae; and 0=mortality. The smaller the number, the higher the B.t. activity. The results are shown in Table I. It is evident that EMCC0075 and EMCC0076 possess activity against both lepidopteran and coleopteran pests.

**TABLE I**

| | *Spodoptera exigua* | *Diabrotica undecimpunctata* | *Heliothis virescens* |
|---|---|---|---|
| EMCC0075 | 1.7 | 0.9 | 1.5 |
| EMCC0076 | 1.8 | 1.8 | 1.8 |
| Control | 4.0 | 4.0 | 4.0 |

### 6.3. EXAMPLE 3: cry GENE PROFILE FOR EMCC0075 AND EMCC0076

The *cry* gene profile for EMCC0075 and EMCC0076 is determined by using the PCR method which is described in the Perkin Elmer Cetus Gene Amp® PCR Reagent Kit literature. Double-stranded DNA is heat-denatured and the two oligonucleotides corresponding to the *cryIA(a)* gene (listed in the Sequence Listing as SEQ ID NO:3 and SEQ ID NO:4 respectively), *cryIA(b)* gene (listed in the Sequence Listing as SEQ ID NO:5 and SEQ ID NO:6 respectively), *cryIA(c)* gene (listed in the Sequence Listing as SEQ ID NO:7 and SEQ ID NO:8 respectively), *cryID* gene (listed in the Sequence Listing as SEQ ID NO:9 and SEQ ID NO:10 respectively), *cryIIIA* gene (listed in the Sequence Listing as SEQ ID NO:11 and SEQ ID NO:12 respectively), *cryIIIB* gene (listed in the Sequence Listing as SEQ ID NO:13 and SEQ ID NO:14 respectively), *cryIIIC* gene (listed in the Sequence Listing as SEQ ID NO:15 and SEQ ID NO:16 respectively), and *cryIIID* gene (listed in the Sequence Listing as SEQ ID NO:17 and SEQ ID NO:18 respectively), are annealed at low temperature and then extended at an intermediate temperature.

PCR analysis indicated that both strains contain a *cryID*-like gene. A probe specific to *cryID* also detected a *cryID*-like gene in Southern analysis of restricted genomic DNA from both strains. No PCR amplifications are observed with primers to *cryIA(a), cryIA(b), cryIA(c), cryIB* (SEQ ID NOS:22 and 23), *cryIC* (SEQ ID NOS:24 and 25), *cryID, cryIE* (SEQ ID NOS:26 and *27*), *cryIF* (SEQ ID NOS:28 and 29), or *cryIG* (SEQ ID NOS:30 and 31), nor to *cryIIA* (SEQ ID NOS:32 and 33), *cryIIB* (SEQ ID NOS:34 and 33), or *cryIIC* (SEQ ID NOS: 35 and 36), nor to *cryIIIA, cryIIIB, cryIIIC,* or *cryIIID*. Southern analysis of a restriction fragment from genomic DNA from EMCC0075 and EMCC0076 with a probe that can detect *cryIA(a), cryIA(b)*, and *cryIA(c)* confirmed the presence of a *cryIA-*like gene.

### 6.4. EXAMPLE 4: PURIFICATION OF EMCC0075 BIPYRAMIDAL AND RHOMBOIDAL CRYSTALS

A subculture of EMCC0075, maintained on a Nutrient Broth agar plate, is used to inoculate a 2.0 liter baffled shake flask containing 500 ml of medium with the same composition as described in Example 5, *infra.* After inoculation, the shake flask is incubated at 30°C on a rotary shaker for 72 hours at 250 rpm. The crystals and spores are recovered by centrifugation at 10,000 rpm (Sorvall GSA rotor) for 30 minutes. The pellets are washed with deionized water, centrifuged at 15,000 rpm (Sorvall SS34 rotor), and resuspended in deionized water by sonication to a concentration of 0.1 g wet weight per ml. 1 g wet weight crude crystals are diluted to 33.2 ml with deionized water and placed in a 250 ml separatory funnel. The bottom phase solution comprised of 10 ml 3M sodium chloride, 23.4 ml 20% polyethylene glycol 8000, and 33.4 ml 20% sodium dextran sulfate is added to the 250 ml separatory funnel and mixed, followed by 100 ml of a polyethylene glycol upper phase solution comprised of 0.3 g sodium dextran sulfate, 70.3 g polyethylene glycol 8000, and 17.5 g sodium chloride per liter deionized water. The suspension is shaken vigorously, and the two phases are allowed to separate at room temperature for 30 minutes.

The upper phase which contains large quantities of spores is removed with a pipet. The lower phase contains crystals and residual spores. The extraction is repeated several times until the upper phase contains essentially no spores. The lower phase is then diluted with 100 ml deionized water, and centrifuged at 10,000 rpm (Sorvall GSA rotor) for 45 minutes at 5°C to recover the crystals. The recovered crystals are washed with 200 ml deionized water, and recentrifuged as before. The spores from the upper phase are also recovered using the above washing procedure.

The bipyramidal and rhomboidal crystals are then further purified by density gradient centrifugation using a discontinuous Ludox™HS-40 (DuPont) gradient comprised of 3.8 ml each of 75%, 50%, and 38% Ludox™ v/v adjusted to pH 2.5 with 0.2M Tris-HCl. 10 mg of crystals in 100 µl deionized water are layered on the top of the gradient, and centrifuged in a Beckman Ultracentrifuge at 10,000 rpm (Beckman 41 Ti rotor) for 15 minutes at 20°C. Four separate bands are obtained. One contains pure rhomboidal crystals and another contains pure bipyramidal crystals. The two other bands contains mixtures of the two crystal types. The pure crystal bands are recovered, washed with deionized water, and used for bioassay.

### 6.5. EXAMPLE 4: SDS-PAGE ANALYSIS OF THE DELTA-ENDOTOXINS FROM EMCC0075 and EMCC0076

Subcultures of EMCC0075 and EMCC0076, maintained on Nutrient Broth agar plates, are used to inoculate 250 ml baffled shake flasks containing 50 ml of medium with the following composition:

| | |
|---|---|
| Glucose | 2.0 g/l |
| KH₂PO₄ | 0.86 g/l |
| K₂HPO₄ | 0.55 g/l |
| Sodium Citrate | 2.0 g/l |
| CaCl₂ | 0.1 g/l |
| MnCl₂·4H₂O | 0.16 g/l |
| MgCl₂·6H₂O | 0.43 g/l |
| ZnCl₂ | 0.007 g/l |
| FeCl₃ | 0.003 g/l |
| Casamino Acids | 5 g/l |

After inoculation, the shake flasks are incubated at 30°C on a rotary shaker for 72 hours at 250 rpm. The B.t. crystals obtained in the above fermentations of EMCC0075 and EMCC0076 are recovered by centrifugation at 10,000 rpm (Sorvall GSA rotor) for 30 minutes. The B.t. crystals are then purified by biphasic extraction using sodium dextran sulfate and polyethylene glycol as outlined in Example 4, *supra.*

B.t. crystal preparations from EMCC0075 and EMCC0076 are analyzed by SDS-PAGE. Specifically, the SDS-PAGE is carried out on 10-15% gradient gels using Pharmacia's Phast System™. The protein bands are analyzed on a Pharmacia densitometer using Pharmacia Gelscan™ Software. The results indicated that the crystals produced by both strains contain at least two proteins with molecular weights of approximately 130,000 daltons and 33,000 daltons.

### 6.6. EXAMPLE 6: BIOASSAY USING SPODOPTERA EXIGUA TO DETERMINE ACTIVITY OF NOVEL LEPIDOPTERAN ACTIVE Bacillus thuringiensis STRAINS

To determine if purified bipyramidal and rhomboidal crystals are active against lepidopteran pests, the crystals are bioassayed against *Spodoptera exigua.* Samples of crystal preparations are applied to individual wells of a jelly tray containing 500 µl of solidified artificial insect diet per well. The trays containing the various samples are air dried. Two to four 2nd or early 3rd instar *Spodoptera* exigua are added to each well containing the dried test sample. The trays are then sealed with Mylar punched with holes for air exchange and are incubated for 3 days at 30°C. The degree of stunting, as described in Example 2, *supra,* is then recorded.

The results are shown in Table II. It is evident that, surprisingly, both the bipyramidal crystal and the rhomboidal crystal possess activity against *Spodoptera exigua*. The spores also show activity against *Spodoptera exigua*.

**TABLE II**

| Sample | Wet Weight | Stunt score |
|---|---|---|
| No crystals or spores | - | 4 |
| Rhomboidal & bipyramidal | 2.5 mg/well | 1 |
| crystals and spores | 5.0 mg/well | 0-1 |
| | | |
| Both crystals, no spores | 2.5 mg/well | 1 |
| | 10 mg/well | 0-1 |
| | | |
| Bipyramidal crystals | 0.092 mg/well | 1 |
| | 0.48 mg/well | 0-1 |
| | | |
| Rhomboidal crystals | 0.05 mg/well | 1 |
| | 0.1 mg/well | 0-1 |
| | 0.5 mg/well | 0 |
| | | |
| Spores | 10 mg/well | 0-1 |
| | 20 mg/well | 0 |

### 6.7. EXAMPLE 7: BIOASSAY USING DIABROTICA UNDECIMPUNCTATA

To determine whether the purified bipyramidal and rhomboidal crystal preparation is active against coleopteran pests, the crystals are bioassayed using a surface overlay assay. Samples of crystal preparations are applied to individual wells of a microtiter plate containing 200 µl of solidified artificial insect diet per well, and then air dried. Two to four neonates of *Diabrotica undecimpunctata* are gently placed in each well with a paintbrush. Then the microtiter plates are sealed with Mylar punched with holes for air exchange and are incubated at 30°C and 80% humidity. Scoring for per cent mortality is carried out at 5 days.

The results are shown in Table III. It is evident that the combination of the bipyramidal crystal and the rhomboidal crystal possesses activity against *Diabrotica undecimpunctata*. The spores alone also have activity against *Diabrotica undecimpunctata*.

**TABLE III**

| Sample | Wet Weight | % Mortality |
|---|---|---|
| No crystals or spores | - | 0% |
| Rhomboidal & bipyramidal crystals and spores | 4 mg/well | 33% |
| Both crystals | 4 mg/well | 88-100% |
| Spores alone | 4 mg/well | 50% |

### 6.8. EXAMPLE 8: PROTEIN SEQUENCING OF THE DELTA-ENDOTOXINS FROM THE RHOMBOIDAL CRYSTAL PROTEINS OF EMCC0075

60 µl of 50% trifluoroacetic acid (TFA) are added to 25 µg of rhomboidal crystals. Four 15 µl aliquots of the mixture are spot dried onto a Biobrene-coated and TFA-pretreated microcartridge glass fiber filter. N-terminal sequencing is performed on a Applied Biosystems Inc. Protein Sequencer Model 476A with on-line HPLC and liquid phase TFA delivery. HPLC determination of phenylthiohydantoin-amino acids is achieved by using the Premix buffer system (ABI Inc.). Data is collected on a Macintosh IIsi using ABI's 610 data analysis software.

A double sequence is observed at approximately a 60/40 ratio. Data are analyzed and the sequences are sorted as follows:

"MIVDL":MIVDLYRYLGGLAAVNAVLHFYEPRP (SEQ ID NO:1)

"MKHHK": MKHHKNFDHI (SEQ ID NO:2)

### 6.9. EXAMPLE 9: CLONING OF THE GEHES ENCODING THE "MIVDL" AND "MKHHK" PROTEINS"

The amino acid sequence initially determined for the "MIVDL" protein, MIVDLYRYLGGLAAVNAVLHFYEPRP, is encoded by the sequence ATG ATH GTN GAY YTN TAY MGN TAY YTN GGN GGN YTN GCN GCN GTN AAY GCN GTN YTN CAY TTY TAY GAR CCN MGN CCN (SEQ ID NO:19). Based on this sequence, a 71 nt oligomer is designed, where mixed deoxynucleotides are used at the 2-fold redundant positions and deoxyinosine at the 4-fold redundant positions to decrease both base discrimination at mismatches and selectivity at incorrect bases (Martin, F. H., and M. M. Castro, 1985, Nucleic Acids Res. 13: 892-8938): ATG ATI GTI GAY YTI TAY MGI TAY YTI GGI GGI YTI GCI GCI GTI AAY GCI GTI YTI CAY TTY TAY GAR CC (SEQ ID NO:20).

The amino acid sequence determined for the "MKHHK" protein, namely, MKHHKNFDHI, permitted design of a more discriminating probe because of the absence of amino acids specified by more than two codons. Further discrimination is permitted by the assumption that As or Ts would be used in the coding sequence in preference to Gs or Cs, due to the overall low % G + C content of B. t. strains (approx 34 moles %, Claus, D., and R. C. W. Berkeley. 1986. Genus *Bacillus, p.* 1112. *In* P. H. A. Sneath (ed.), Bergey's manual of systematic bacteriology, v. 2. The Williams and Wilkins Co., Baltimore). The following probe is synthesized: ATG AAA CAT AAA AAT TTT GAT CAT AT (SEQ ID NO:21). Both the MIVDL and the MKHHK probes are tailed with digoxygenin-dUTP according to the manufacturer's instructions (Boerhinger-Mannheim Genius System™ Users Guide, Version 2.0).

EMCC0075 genomic DNA is digested with *Eco*RI, *Eco*RV, *Hin*dIII, *Pst*I, or combinations of those enzymes overnight in buffers supplied by the manufacturers, electrophoresed through 0.8% agarose in 0.5X TBE (TRIS-borate-EDTA buffer; Sambrook et al., 1989, in Molecular Cloning, a Laboratory Manual, Cold Spring Laboratory Press, Cold Spring Harbor, N.Y.), transferred in 10X SSC to Boehringer Mannheim nylon membrane with a Stratagene Posiblotter in 10X SSC, and then probed as described below. The MIVDL probe, after hybridization and stringent washing at 48°C with 0.5X SSC, detected *Eco*RV and *Pst*I fragments 12 kb or more in size, an *Eco*RI fragment of approx 10 kb, and a *Hin*dIII fragment of approx 3.5 kb. The MKHHK probe, after hybridization and stringent washing at 48°C with 5 X SSC, detected the same size *Eco*RI, *Eco*RV, and *Pst*I fragments as did the MIVDL probe. This result indicates that the two genes probably lie in close proximity to each other. Additionally, the MKHHK probe detected a *Hin*dIII fragment of approx 6 kb.

To clone the *Hind*III fragments encoding at least part of the "MIVDL" and "MKHHK" proteins, pUC118 is digested with *Hind*III, and then treated with calf intestinal phosphatase to dephosphorylate the 5' ends and thus prevent vector religation. Restricted and phosphatased pUC118 is then mixed with EMCC0075 genomic DNA that had been previously digested to completion with *Hind*III. After ligation, the reaction mix is used to transform E. *coli* strain XL1-Blue MRF' (Stratagene, Inc., La Jolla, CA). Colonies harboring the desired DNA fragment are detected by "colony hybridization" with the aforementioned "MIVDL" and "MKHHK" probes by the procedure described by Sambrook et al., 1989, Molecular cloning, a Laboratory Manual. Cold Spring Laboratory Press, Cold Spring Harbor, N.Y.

### 7. DEPOSIT OF MICROORGANISMS

The following strains of *Bacillus thuringiensis* have been deposited in the Agricultural Research Service Patent Culture Collection (NRRL), Northern Regional Research Center, 1815 University Street, Peoria, Illinois, 61604, USA.

| Strain | Accession Number | Deposit Date |
|---|---|---|
| EMCC0075 | NRRL B-21019 | December 3, 1992 |
| EMCC0076 | NRRL B-21020 | December 3, 1992 |

The strains have been deposited under conditions that assure that access to the culture will be available during the pendency of this patent application to one determined by the Commissioner of Patents and Trademarks to be entitled thereto under 37 C.F.R. §1.14 and 35 U.S.C. §122 and under conditions of the Budapest Treaty. The deposit represents a biologically pure culture of each deposited strain. The deposit is available as required by foreign patent laws in countries wherein counterparts of the subject application, or its progeny are filed. However, it should be understood that the availability of a deposit does not constitute a license to practice the subject invention in derogation of patent rights granted by governmental action.

The invention described and claimed herein is not to be limited in scope by the specific embodiments herein disclosed, since these embodiments are intended as illustrations of several aspects of the invention. Any equivalent embodiments are intended to be within the scope of this invention. Indeed, various modifications of the invention in addition to those shown and described herein will become apparent to those skilled in the art from the foregoing description. Such modifications are also intended to fall within the scope of the appended claims.

Various references are cited herein, the disclosures of which are incorporated by reference in their entireties.

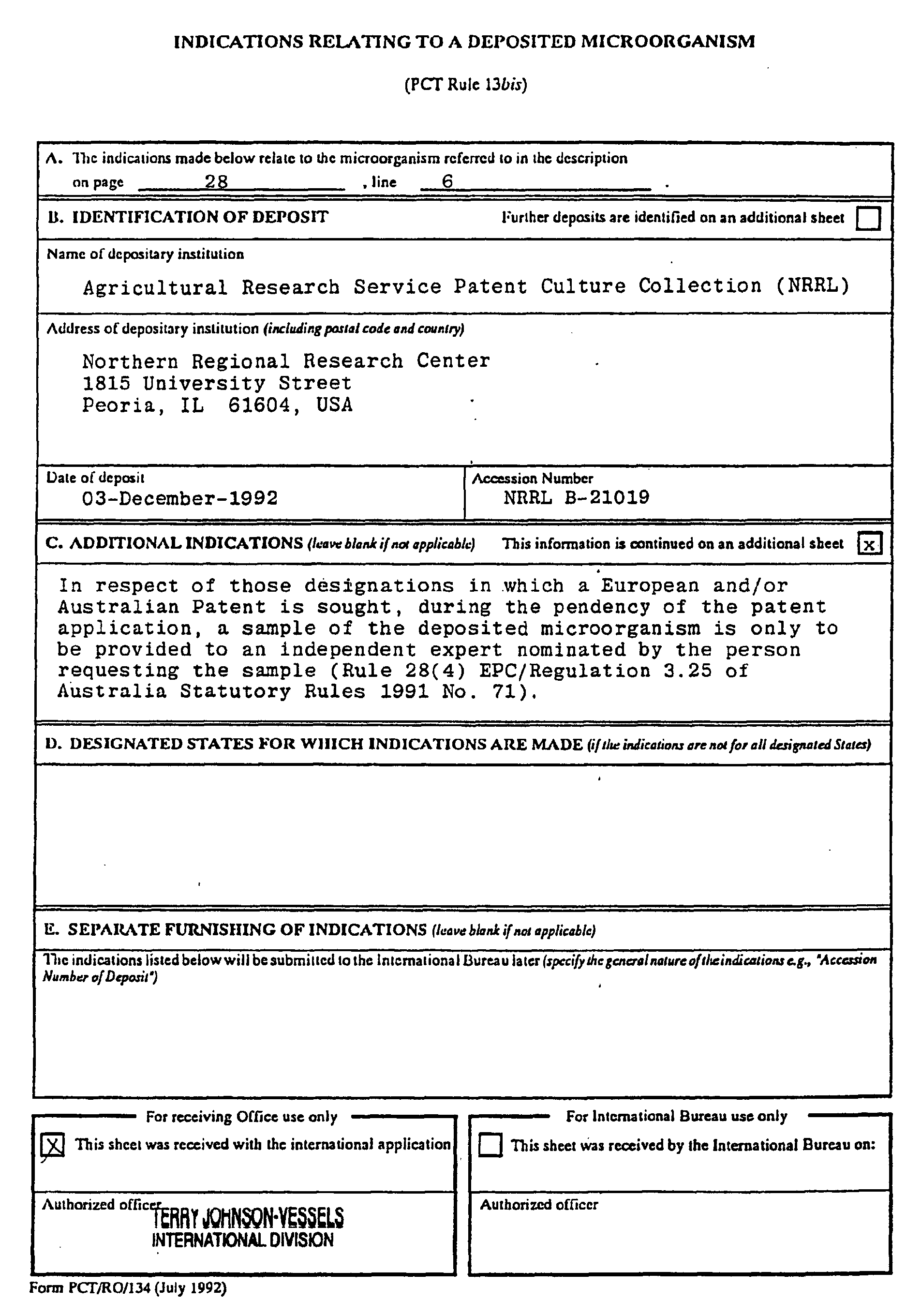

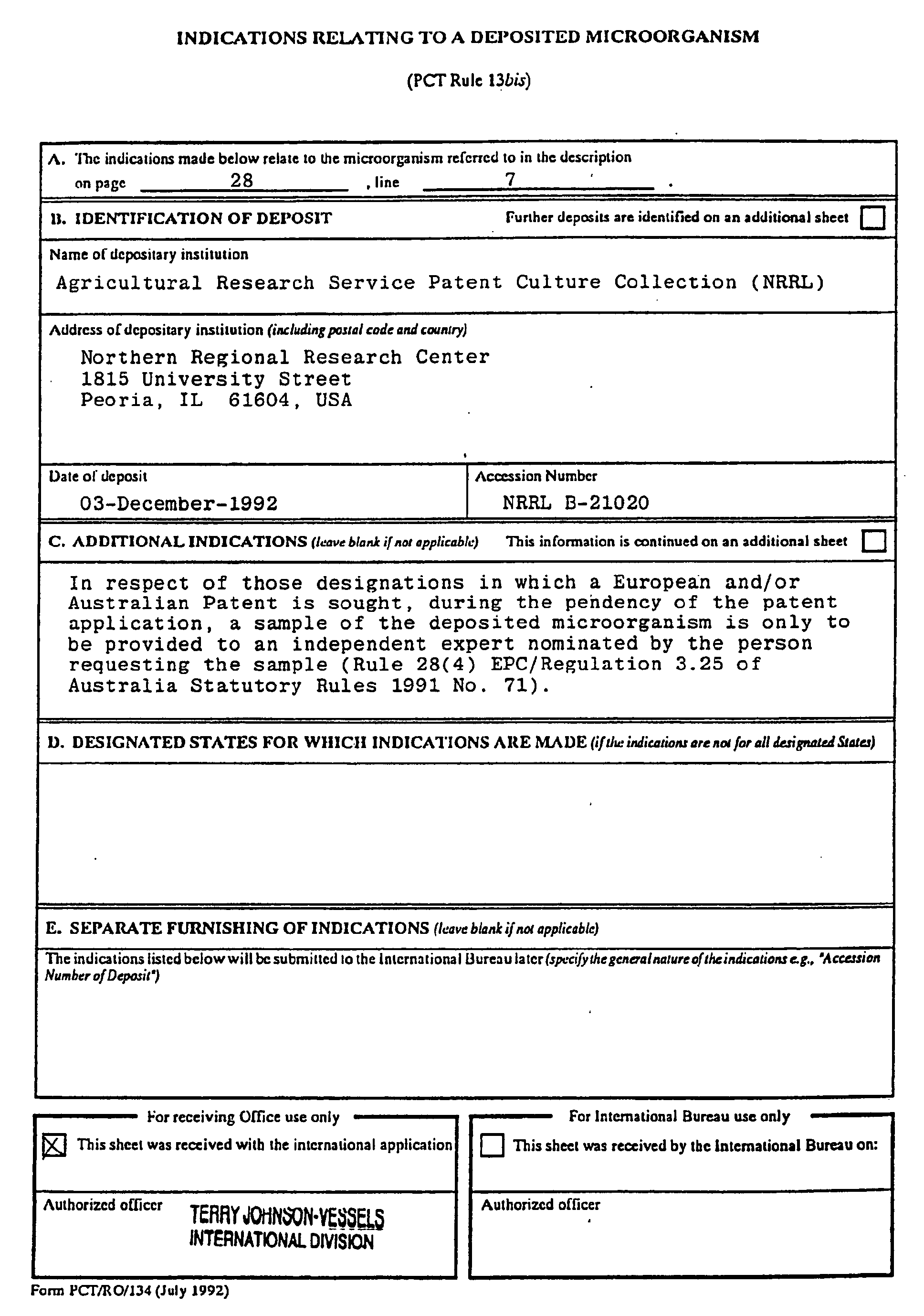

## Claims

1. A delta-endotoxin having a molecular weight of about 33,000 daltons and an N-terminal amino acid sequence of

MIVDLYRYLGGLAAVNAVLHFYEPRP (SEQ ID NO:1).

2. The delta-endocoxin of claim 1 in which the delta-endotoxin is obtained from *Bacillus churingiensis* EMCC0075 having the identifying characteristics of NRRL B-21019, or a spore or mutant thereof which have substantially the same properties as *Bacillus thuringiensis* EMCC0075 or *Bacillus thuringiensis* EMCC0076 having the identifying characteristics of NRRL B-21020, or a spore or mutant thereof which have substantially the same properties as *Bacillus thuringiensis* EMCC0076.

3. A delta-endotoxin having a molecular weight of about 33,000 daltons and an N-terminal amino acid sequence of

MKHHKNFDHI (SEQ ID NO:2).

4. The delta-endotoxin of claim 3 in which the delta-endotoxin is obtained from *Bacillus thuringiensis* EMCC0075 having the identifying characteristics of NRRL B-21019, or a spore or mutant thereof which have substantially the same properties as *Bacillus thuringiensis* EMCC0075 or *Bacillus thuringiensis* EMCC0076 having the identifying characteristics of NRRL B-21020, or a spore or mutant thereof which have substantially the same properties as *Bacillus thuringiensis* EMCC0076.

5. A nucleic acid fragment containing a nucleic acid sequence encoding a delta-endotoxin having a molecular weight of about 33,000 daltons and an N-terminal amino acid sequence of MIVDLYRYLGGLAAVNAVLHFYEPRP (SEQ ID NO:1) or a portion of said delta-endotoxin having insecticidal activity against an insect pest of the order Lepidoptera or Coleoptera.

6. A nucleic acid fragment containing a nucleic acid sequence encoding a delta-endotoxin having a molecular weight of about 33,000 daltons and an N-terminal amino acid sequence of MKHHKNFDHI (SEQ ID NO:2) or fragment thereof encoding a portion of said delta-endotoxin having insecticidal activity against an insect pest of the order Lepidoptera or Coleoptera.

7. An insecticidal composition comprising a delta-endotoxin having a molecular weight of about 33,000 daltons and an N-terminal amino acid sequence of MIVDLYRYLGGLAAVNAVLHFYEPRP (SEQ ID NO:1) and a delta-endotoxin having a molecular weight of about 33,000 daltons and an N-terminal amino acid sequence of MKHHKNFDHI (SEQ ID NO:2), in association with an insecticidal carrier.

8. A method for controlling an insect pest of the order Lepidoptera comprising exposing the pest to an insect-controlling effective amount of an insecticidal composition of claim 7,
